# EUROPÄISCHE PATENTSCHRIFT

(11) **EP 2 331 148 B1**
(45) Veröffentlichungstag und Bekanntmachung des Hinweises auf die Patenterteilung: **26.02.2014**
(21) Anmeldenummer: 09777620.7
(22) Anmeldetag: 04.08.2009
(51) Int. Cl.: A61L 2/20, A61L 2/26, B67B 3/00

(54) **VORRICHTUNG ZUM STERILISIEREN VON VERSCHLÜSSEN**
DEVICE FOR STERILIZING CLOSURES
DISPOSITIF DE STÉRILISATION DE FERMETURES

(30) Priorität: 22.09.2008 DE 102008048351
(43) Veröffentlichungstag der Anmeldung: 15.06.2011
(73) Patentinhaber: KHS GmbH, 44143 Dortmund (DE)
(72) Erfinder: BECKMANN, Jörg, 21680 Stade (DE); DRENGUIS, Alfred, 21039 Börnsen (DE)
(86) Internationale Anmeldenummer: PCT/EP2009/005614
(87) Internationale Veröffentlichungsnummer: WO 2010/031464

(56) Entgegenhaltungen:
- EP-A- 1 749 747
- DE-A1- 10 145 102
- DE-A1- 19 727 942
- FR-A1- 2 789 065
- JP-A- 2003 128 023
- US-A- 4 296 769

## Beschreibung

Die Erfindung bezieht sich auf eine Vorrichtung gemäß Oberbegriff Patentanspruch 1.

Eine Vorrichtung dieser Art ist bekannt (DE 101 45 102 A1). Bei der bekannten Vorrichtung werden die in einen Förderkanal eingebrachten Verschlüsse oder Kappen durch ein von einem Kappenrad gebildetes Förderelement dicht aneinander anschließend in einem einspurigen Kappen- oder Verschlussstrom durch den Förderkanal geschoben. Am Förderkanal sind in Bewegungsrichtung der Verschlüsse aufeinander folgend und voneinander beabstandet mehrere Behandlungsstationen in Form von Düsanstöcken vorgesehen. Nachteil sind u.a. die relativ geringe Leistung (Anzahl der behandelten bzw. sterilisierten Verschlüsse je Zeiteinheit) sowie die Gefahr eines Verklemmens der Verschlüsse im Förderkanal.

Bekannt ist weiterhin eine Vorrichtung zum Sterilisieren von Verschlüssen (DE 103 59 392 B3), bei der die Verschlüsse über mehrere, jeweils von einer umlaufend angetriebenen Scheibe gebildete Etagen und über die Etagen miteinander verbindende schiefe Ebenen von oben nach unten durch einen mit einem Desinfektions- oder Sterilisationsmedium beaufschlagten Behandlungsraum bewegt werden. Diese bekannte Vorrichtung ist konstruktiv aufwendig, bedingt eine relativ große Bauform und Ist darüber hinaus hinsichtlich möglicher Sterilisationsverfahren stark eingeschränkt.

Die Druckschrift US 4 296 769 A offenbart eine Vorrichtung zur Behandlung von Verschlüssen. Die Vorrichtung weist zwei parallel zueinander verlaufende, umlaufend angetriebene Ketten auf, die an ihren einander zugewandten Kettenseiten Aufnahmen für Verschlusskörbe aufweisen. Die Verschlusskörbe bestehen aus mehreren parallel zueinander verlaufenden, beabstandeten, stangenförmigen Elementen, die zur Aufnahme mehrerer Verschlusse in einer Reihe ausgebildet sind. Nach der Bestückung der Körbe mit Verschlüssen werden diese in die Aufnahmen der Ketten eingelegt und damit durch die Ketten entlang einer Behandlungsstrecke transportiert und mit Sterilisationsmittel besprüht.

Druckschrift EP 1 749 747 A offenbart eine Vorrichtung zur Verarbeitung von Verschlüssen. Die Vorrichtung besteht aus mehreren rotierenden Zylindern die zur Aufnahme und zum Transport der Verschlüsse ausgebildet sind. Die Zylinder welsen umfangsseltig Ausnehmungen auf, die zur Aufnahme der Oberseite bzw. der Unterseite des Verschlusses ausgebildet sind. Die jeweils aufeinander folgenden Zylinder sind hierbel gegenläufig zueinander angetrieben, sodass die dem ersten Zylinder zugeführten Verschlüsse von einer Verschlusszuführungsposition mittels der jeweiligen Ausnehmung aufgenommen und an eine Übergabeposition zu dem weiteren, darauf folgenden Zylinder weitertransportiert werden. An dieser Übergabeposition werden die Verschlüsse an den darauf folgenden Zylinder weitergegebenen, sodass die Verschlüsse abwechselnd mit ihren Ober- bzw. Unterseiten in den Ausnehmungen der Zylinder gehalten werden. Hierbei ist es bereits bei der Zuführung der Verschlüsse unbedingt notwendig, dass die Verschlüsse die erforderliche Orientierung aufweisen, um mit den Ausnehmungen des ersten, auf die Verschlusskappenzuführung folgenden Zylinders zusammen wirken können.

Die Druckschrift FR 2 789 065 A1 zeigt eine trommelartige Sterilisationsvorrichtung für Verschlüsse. Die zugeführten Verschlüsse werden hierbei in einer spiralförmig verlaufenden Führung relbungsschlüssig mit zumindest zwei gegenüberliegenden Wandabschnitten bewegt und während der Bewegung durch die spiralförmige Führung mehreren Behandlungsstationen unterzogen, insbesondere sterilisiert, gespült und getrocknet. Aufgrund der spiralförmigen Führung und des Reibungsschlusses ist eine Sortierung der Verschlüsse innerhalb der Vorrichtung nicht vorgesehen und auch nicht möglich.

Aufgabe der Erfindung ist es, eine Vorrichtung aufzuzeigen, die bei kompakter Bauweise eine Sterilisation von Verschlüssen oder Kappen bei optimaler Qualität mit hoher Leistung ermöglicht. Zur Lösung dieser Aufgabe ist eine Vorrichtung entsprechend dem Patentanspruch 1 ausgebildet.

Die erfindungagemäße Vorrichtung ist flexibel hinsichtlich der verwendeten Sterilisationsverfahren, d.h. sie ist für unterschiedlichste Sterilisationsverfahren für die Sterilisation von Kappen oder anderen Verschlüssen zum Verschließen von Flaschen oder dergleichen Behältern geeignet. Dennoch eignet sich die erfindungsgemäße Vorrichtung in besonders vorteilhafter Weise für eine Sterilisation von Kappen oder anderen Verschlüssen unter Verwendung eines Wasserstoffperoxid (H2O2) enthaltenden Sterilisationsmedium durch Aufbringen dieses Sterilisationsmediums auf die Verschlüsse in einer Applikationsphase und durch anschließendes Aktivieren des Wasserstoffperoxids in einer Aktivierungsphase durch Energieeintrag, beispielsweise durch Erwärmen, z.B. durch Beaufschlagung der Verschlüsse mit einem heißen gas- und/oder dampfförmigen Aktivierungsmedium, beispielsweise mit heißer steriler Luft zum Freisetzen der die Sterilisation bewirkenden Verschlüsse, beispielsweise unmittelbar vor der Sterilisation hergestellte Verschlüsse, die noch mit der Produktionstemperatur der Herstellung zugeführt werden, auf die Temperatur oder Oberflächentemperatur abgekühlt werden.

Durch das Temperieren der Verschlüsse in der Vorbehandlungsphase, beispielsweise durch Beaufschlagung mit einem heißen sterilen gas- und/oder dampfförmigen Medium, z.B. mit heißer steriler Luft oder auf andere Weise, z.B. durch Strahlungswärme, ergibt sich in ganz überraschender Weise eine deutliche Verkürzung der gesamten Behandlungsdauer, und zwar einschließlich der Dauer der Vorbehandlung bzw. des Temperierens, der Applikationsphase und der Aktivierungs- und Trocknungsphase. Es hat sich gezeigt, dass ohne Beeinträchtigung der Qualität der Sterilisation bzw. der Entkeimungsrate die Gesamtbehandlungszeiten auf 15 - 10 sec., beispielsweise auf ca. 12 sec. verkürzt werden kann, und zwar bei einer Dauer von etwa jeweils 5 sec. für das Temperieren und für die Applikationsphase und bei einer Dauer von nur 2 sec. oder etwa 2 sec. für die Aktivierungs- und Trocknungsphase. Überraschend ist hierbei insbesondere die extreme Verkürzung der Behandlungsdauer für die Aktivierungs- und Trocknungsphase, obwohl diese sich nicht unmittelbar an die Vorhandlungsphase anschließt.

Die einzelnen Verfahrensschritte bzw. Behandlungsphasen sind so gesteuert, dass die Oberflächentemperatur der Verschlüsse einen oberen Grenzwert, beispielsweise einen Grenzwert im Bereich von 70°C oder etwa 70°C nicht übersteigt. Speziell bei Verschlüssen, die vollständig oder teilweise aus Kunststoff bestehen, wird hierdurch eine Beschädigung der Verschlüsse durch eine zu hohe thermische Belastung vermieden.

Nach dem Aktivieren und Trocknen erfolgt dann beispielsweise ein Abkühlen der Verschlüsse in einer Abkühlphase, und zwar auf eine Temperatur deutlich unter 70°C, beispielsweise auf eine Temperatur von 30°C oder etwa 30°C oder tiefer, so dass die Verschlüsse dann mit guten und insbesondere auch konstanten bzw. reproduzierbaren Eigenschaften für die weitere Verwendung bzw. für die Verarbeitung in einer Verschließmaschine zur Verfügung stehen.

Durch die stark verkürzte Gesamtbehandlungszeit ergibt sich eine erhebliche Einsparung von Betriebskosten sowie insbesondere aber auch der Vorteil, dass die erfindungsgemäße Vorrichtung, in der die Sterilisation der Verschlüsse im Durchlaufverfahren durchgeführt wird, kompakt und mit geringer Baugröße realisiert werden können.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:
- Fig. 1: in schematischer perspektivischer Darstellung und teilweise geöffnet eine Vorrichtung zum Sterilisieren von Verschlüssen für Flaschen oder dgl. Behälter;
- Fig. 2: in schematischer Darstellung und in Seitenansicht die Sterilisationsstrecke der Vorrichtung der Fig. 1;
- Fig. 3: einen der Kappenkörbe des Transportelementes der Vorrichtung der Fig. 1 und 2;
- Fig. 4 und 5: jeweils in sehr schematischer Darstellung die Sterilisationsstrecke der Vorrichtung der Fig. 1 - 3 bei unterschiedlichen Ausführungen, und zwar zusammen mit mehreren an dieser Behandlungsstrecke vorgesehenen Behandlungszonen;
- Fig. 6: in sehr schematischer Darstellung eine Ausführungsform;
- Fig. 7 und 8: in perspektivischer Darstellung eine Trommel zum Sterilisieren von Verschlüssen für Flaschen oder dergleichen Behälter;
- Fig. 9: die Trommel der Figuren 7 und 8 in schematischer Darstellung.

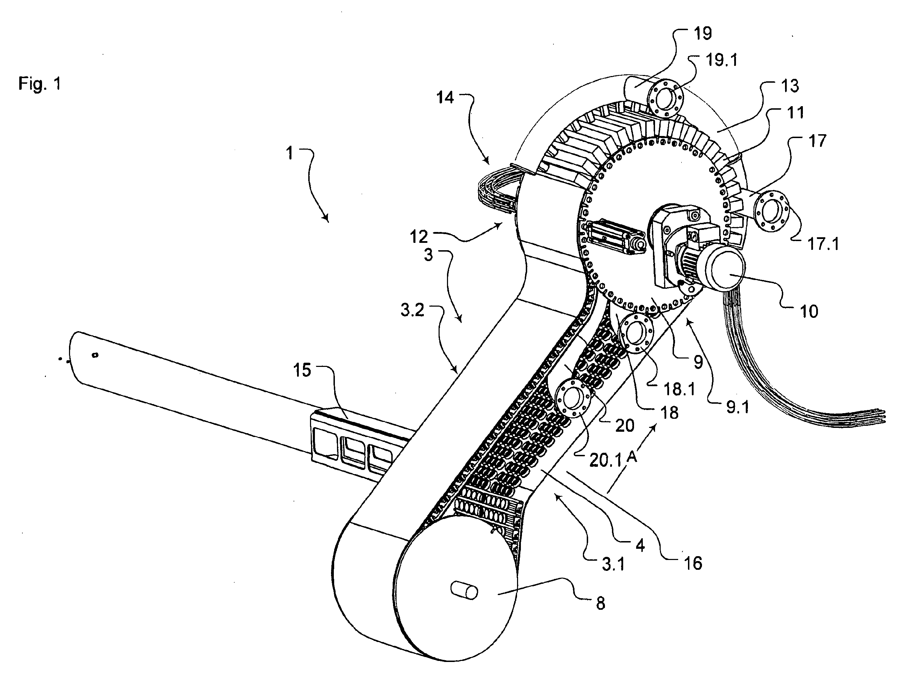

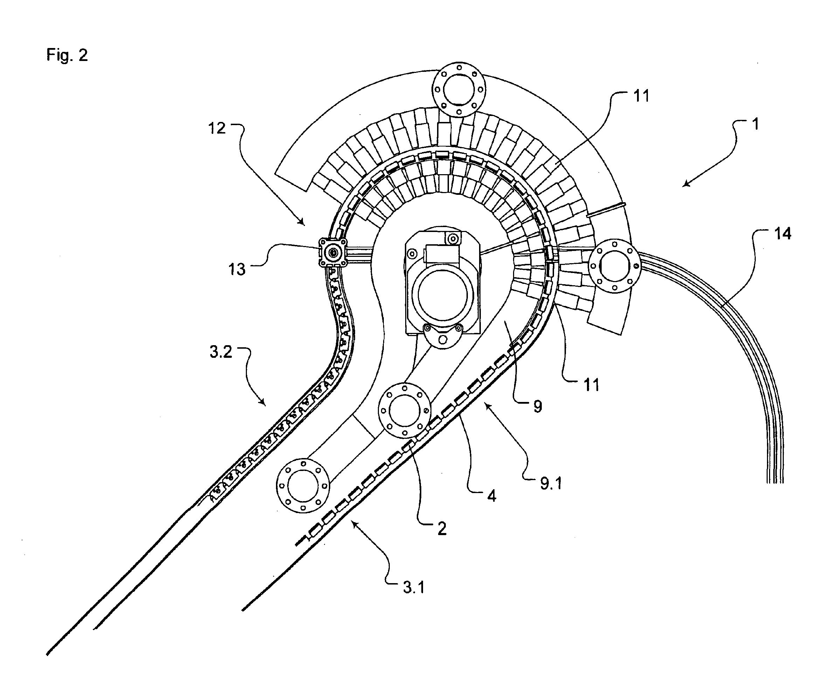

Die in den Fig. 1 - 5 allgemein mit 1 bezeichnete Vorrichtung dient zum Sterilisieren von Verschlüssen in Form von Kappen 2 vor dem Zuführen dieser Kappen an eine nicht dargestellte Verschließmaschine zum Verschließen von Flaschen oder dgl. Behältern. Die Kappen 2 sind beispielsweise solche, die zumindest teilweise aus Kunststoff gefertigt und/oder als Schraubkappen ausgebildet sind.

Bestandteil der Vorrichtung 1 ist ein Transporteur oder Transportelement 3 mit einem eine geschlossene Schlaufe bildenden und endlos umlaufend antreibbaren Transportband 4, welches gliederkettenartig von einer Vielzahl von Kappenkörben 4.1 gebildet ist, die gelenkig miteinander verbunden sind und mit ihrer Längserstreckung jeweils senkrecht zur Transportrichtung A orientiert sind. Jeder Kappenkorb besteht aus zwei senkrecht zur Transportrichtung A voneinander beabstandeten plattenförmigen Endstücken 5 und aus drei die Endstücke 5 miteinander verbindenden rohr- oder stangenartigen Stegen 6 und 7, von denen die beiden parallel zueinander und voneinander beabstandeten Stege 6 Auflagen für die offene oder geschlossene Seite der napfartigen Kappen 2 bilden. An den Stegen 7 und den Endstücken 5 sind die Kappenkörbe 4.1 gelenkig miteinander verbunden. Jeder Kappenkorb 4.1 bildet eine Aufnahme für mehrere Kappen 2, in denen diese mit ihrer geschlossenen oder offenen Seite an den Stegen 6 aufliegen und seitlich, d.h. in Transportrichtung A und entgegen der Transportrichtung A durch die Stege 7 abgestützt sind. Durch die beschriebene Ausbildung der Kappenkörbe 4.1 sind die Kappen 2 in den Kappenkörben 4.1 allseitig mit leichtem Spiel und weitestgehend freiliegend aufgenommenen. Die einzelnen Kappenkörbe 4.1 sind weiterhin so zu dem Transportband 4 miteinander verbunden, dass sich die Stege 6 an der Außenseite der von dem Transportband 4 gebildeten Schlaufe befinden.

An dem in der Fig. 1 unteren Ende ist das Transportband 4 im Bereich eines dortigen, trommelartigen Bunkers 8 zur Aufnahme der Kappen 2 über eine nicht dargestellte Umlenkung geführt. An dem der Fig. 1 oberen Ende ist die Stege über eine von einer Sterilisationstrommel 9 gebildete Umlenkung geführt, die von einem Elektromotor 10 für die Bewegung des Transportbandes 4 in Transport- oder Förderrichtung A umlaufend antreibbar ist.

An der Sterilisationstrommel 9 sind außerhalb sowie innerhalb der von dem Transportband 4 gebildeten Schlaufe in Transportrichtung A aufeinander folgend mehrere Behandlungsstationen 11 vorgesehen, in denen die Kappen 2 mit wenigstens einem Desinfektions- oder Sterilisationsmedium behandelt werden, beispielsweise in der nachstehend noch näher beschriebenen Weise in mehreren Behandlungsschritten.

Durch ein äußeres Gehäuse sind das Transportelement sowie alle weiteren, mit den Kappen 2 zusammenwirkenden Funktionselemente, insbesondere auch der Bunker 8 und die Behandlungsstationen 11, der Kappenauslass 12 usw. aufgenommen. Die Funktionsweise der Vorrichtung 1 lässt sich, wie folgt beschreiben:

Die Kappen 2 werden über eine Zuführung 15 als ungeordnete Menge in den Bunker 8 eingebracht, aus dem dann bei umlaufendem Transportband 4 mit den zur Innenseite des Bunkers 18 offenen Kappenkörben 4.1 jeweils eine Anzahl von Kappen 2 mitgeführt werden. Durch entsprechende Führungen und/oder Schikanen ist dafür gesorgt, dass die in jedem Kappenkorb 4.1 aufgenommenen Kappen 2 allenfalls nur zwei mögliche Orientierungen aufweisen können, nämlich mit der offenen Kappenseite oder der geschlossenen Kappenseite auf den Stegen 6 der Kappenkörbe 4.1 aufliegend. Durch weitere Maßnahmen, beispielsweise durch entsprechende Ausbildung einer Öffnung, durch die die Kappen 2 aus dem Bunker 8 in die Kappenkörbe 4.1 gelangen und/oder durch entsprechende Schikanen ist auch dafür gesorgt, dass jeweils nur eine, sich senkrecht zur Transportrichtung A erstreckende Teillänge jedes Kappenkorbes 4.1 mit Kappen 2 belegt ist, beispielsweise nur 50% - 70 % der Gesamtlänge jedes Kappenkorbes 4.1. Mit dem umlaufenden Transportband 4 werden die in den Kappenkörben 4.1 aufgenommenen Kappen 2 auf der Förderstrecke 3.1 des Transporteurs zunächst an einer Sortierstation vorbeibewegt, die in der Fig. 1 nur sehr schematisch mit 16 bezeichnet ist und in der solche Kappen 2, die nicht die gewünschte, richtige Orientierung aufweisen, d.h. beispielsweise nicht mit ihrer offenen Kappenseite bezogen auf die von dem Transportband 4 gebildete Schlaufe nach innen weisen, d.h. nicht mit ihrer geschlossenen Kappenseite oder ihrem geschlossenen Kappenboden auf den Stegen 6 aufliegen, ausgeworfen werden. Die richtig orientierten Kappen 2 gelangen dann mit dem Transportband 4 in den Einlauf 9.1 der Sterilisationstrommel 9 und werden anschließend für die Behandlung bzw. Sterilisation an den verschiedenen Behandlungsstationen 11 vorbeibewegt. Es versteht sich, dass die Behandlungsstationen 11 hierfür so ausgebildet sind, dass sie jeweils über die gesamte Länge der Kappenkörbe 4.1 wirksam sind.

Vor dem Erreichen der Behandlungsstationen 11 werden die Kappen 2 in jeden Kappenkorb 4.1 durch geeignete Mittel voneinander beabstandet, z.B. durch Rollen, Verschieben usw., und zwar unter Verwendung von mechanischen Führungen oder Schikanen und/oder durch Einwirken eines die Kappen 2 bewegenden gas- und/oder dampfförmigen Mediums, beispielsweise des in der jeweiligen Behandlungsstation 11 verwendeten Behandlungsmediums. Hierbei besteht insbesondere die Möglichkeit, die Kappen 2 auch während der Behandlung an den verschiedenen Behandlungsstationen 11 und/oder beim Weitertransport von einer Behandlungsstation 11 an die in Transportrichtung A folgende Behandlungsstation innerhalb des jeweiligen Kappenkorbes 4.1 in Kappenkorblängsrichtung zu bewegen, d.h. insbesondere zu verschieben und/oder zu drehen, um so eine Behandlung bzw. Sterilisation der Kappen 2 an ihrer gesamten Außen- und Innenfläche sicherzustellen.

Die behandelten bzw. sterilisierten Kappen 2 gelangen dann nach dem Passieren der Behandlungsstationen 11 an einen Kappenauslass 12, an welchem die in jeweils einem Kappenkorb 4.1 vorhandenen, behandelten bzw. sterilisieren Kappen 2 als Gruppe mit Hilfe einer beispielsweise einen Ausstoßzylinder aufweisenden Ausstoßeinrichtung 13 in einer Achsrichtung senkrecht zur Transportrichtung A und parallel zur Achse der Sterilisationstrommel 9 in einen käfigartigen Kappenkanal 14 ausgestoßen werden, und zwar zum Zuführen an eine Verschließstation einer der Vorrichtung 1 zugeordneten Verschließmaschine. Hierfür ist die Ausstoßeinrichtung 13 synchron mit der getakteten Bewegung des Transportbandes 4 angetrieben. Die leeren Kappenkörbe 4.1 gelangen über die Leerstrecke 3.2 zur erneuten Ausnahme von Kappen 2 an den Bunker 8 zurück. Der käfigartige Kappenkanal 14, in welchem die Kappen 2 allseitig mit leichtem Spiel und weitestgehend freiliegend geführt sind, ist in einem nicht dargestellten, zur Umgebung hin dicht verschlossenen rohrförmigen Gehäuse aufgenommen, welches bevorzugt ebenfalls von einem Sterilisationsmedium durchströmt wird.

Mit der Vorrichtung 1 sind unterschiedlichste Verfahren zum Behandeln bzw. zum Sterilisieren der Kappen 2 möglich. Bei einem bevorzugten Verfahren werden die Kappen 2 im Bunker 8 und/oder auf der Transportstrecke 3.1 temperiert, d.h. so vorbehandelt, dass die in den Kappenkörben 4.1 vorhandenen Kappen 2 bei Erreichen der Sterilisationstrommel 9 bzw. der dort in Transportrichtung A ersten Behandlungsstation 11 eine Vorbehandlungs- oder Oberflächentemperatur unter 70°C, beispielsweise eine Oberflächentemperatur im Bereich zwischen 30°C und 60°C, bevorzugt zwischen 35°C und 40°C aufweisen, und zwar mit Abweichungen von +/-10°C, bevorzugt mit Abweichungen zwischen +/-2°C bis 3°C.

Werden die Kappen 2 dem Bunker 8 mit einer Temperatur unterhalb dieser Oberflächentemperatur zugeführt, so erfolgt das Temperieren durch Vorwärmen der Kappen 2 im Bunker 8 und/oder auf der Förderstrecke 3.1, beispielsweise mit einem heißen, bevorzugt sterilen gas- und/oder dampfförmigen Medium, z.B. mit erwärmter steriler Luft, oder aber auf andere Weise, z.B. durch Strahlungsenergie beispielsweise durch Infrarotstrahlung.

Gelangen die Kappen 2 in den Bunker 8 und aus diesem in die Kappenkörbe 4.1 mit einer Oberflächentemperatur, die deutlich oberhalb der vorgenannten Temperatur liegt, beispielsweise deswegen, weil die Kappen 2 dem Bunker 8 unmittelbar nach ihrer Herstellung zugeführt und daher noch die aus dem Herstellungsverfahren resultierende Produktionstemperatur aufweisen, so kann das Temperieren im Bunker 8 und/oder auf der Transportstrecke 3.1 auch darin bestehen, dass eine Abkühlung der Kappen 2 von der höheren Produktionstemperatur auf die Vorbehandlungstemperatur erfolgt.

An den einzelnen Behandlungsstationen 11 erfolgt dann bei diesem bevorzugten Verfahren zunächst in einer Applikationsphase an einer oder an mehreren in Transportrichtung A aufeinander folgenden Behandlungsstationen 11 das Behandeln der Kappen 2 durch Applizieren eines Wasserstoffperoxid (H2O2) enthaltenden heißen Sterilisationsmediums, welches aus einem Gemisch aus einem erhitzten gas- und/oder dampfförmigen Medium, beispielsweise aus erhitzter steriler Luft und Wasserstoffperoxid (H2O2) in verdampfter Form besteht. Im Anschluss daran erfolgt in einer Aktivierungsphase an einer oder an mehreren in Transportrichtung A aufeinander folgenden Behandlungsstationen 11 ein Aktivieren des auf die Kappen 2 aufgebrachten Sterilisationsmediums durch Beaufschlagung mit einem erhitzten dampf- und/oder gasförmigen Aktivierungsmedium, beispielsweise durch Beaufschlagung mit erhitzter steriler Luft, so dass durch das Aktivieren des im Sterilisationsmedium enthaltenen Wasserstoffperoxids, d.h. durch Abspalten von freien Sauerstoff-Radikalen aus dem Wasserstoffperoxids die Kappen 2 mit der erforderlichen hohen Qualität sterilisiert werden. Nach dem Aktivieren des Sterilisationsmediums erfolgt in der wenigstens einen hierfür verwendeten Behandlungsstation 11 oder aber in einer weiteren Behandlungsstation das Trocknen der Kappen 2 mit dem erhitzten Aktivierungsmedium, oder aber mit erhitzter steriler Luft.

Es hat sich gezeigt, dass durch das Temperieren der Kappen 2 vor dem Zuführen an die wenigstens eine, zum Applizieren bzw. Aufbringen des Sterilisationsmediums dienende Behandlungsstation 11 in völlig überraschender Weise eine deutliche Verkürzung der gesamten Behandlungsdauer, und zwar einschließlich der Dauer der Vorbehandlung bzw. des Temperierens, der Applikationsphase und der Aktivierungsphase sowie der Trocknungsphase erreichbar ist. Es hat sich weiterhin überraschenderweise gezeigt, dass trotz des Temperierens der Kappen 2 eine für das Sterilisieren notwendige Kondensatbildung des in der Applikationsphase aufgebrachten Sterilisationsmediums an allen Oberflächenbereichen der Kappen 2 erreicht wird, insbesondere auch in schwer zugänglichen Oberflächenbereichen, wie Hinterschneidungen usw. Es hat sich weiterhin gezeigt, dass ohne Beeinträchtigung der Qualität der Sterilisation bzw. der Entkeimungsrat die Gesamtbehandlungszeit z.B. auf 15 - 10Sec., beispielsweise auf ca. 12 Sec. verkürzt werden kann, und zwar bei einer Dauer von etwa 5 Sec. für die Applikationsphase und bei einer Dauer von nur 2 Sec. oder etwa 2 Sec. für die Aktivierungs- und Trocknungsphase.

Bevorzugt erfolgt nach dem Trocknen und vor dem Ausbringen der Kappen 2 in dem Kappenkanal 14 noch ein Abkühlen der Kappen 2 auf eine Oberflächentemperatur deutlich unter 70°C, beispielsweise auf eine Oberflächentemperatur unter 50°C, z.B. auf eine Oberflächentemperatur von etwa 30°C, um so auch für aus Kunststoff gefertigte Kappen 2 die insbesondere beim Verschließen notwendige Festigkeit zu erreichen, und zwar bevor die Kappen 2 der Verschließmaschine bzw. deren Verschließwerkzeuge zugeführt werden. Das Abkühlen der Kappen 2 kann auch im Kappenkanal 14 erfolgen, beispielsweise mit Hilfe eines kühlenden sterilen gas- und/oder dampfförmigen Mediums, z.B. mit kühler steriler Luft, die das den Kappenkanal 14 aufnehmende rohrförmige Gehäuse durchströmt.

Zusätzlich zu der Reduzierung der Gesamtbehandlungsdauer ergibt sich bei diesem Verfahren in überraschender Weise auch eine Reduzierung der Betriebskosten sowie auch eine Reduzierung der Baugröße der Vorrichtung 1 insbesondere im Bereich der Behandlungsstationen 11. Bei Füll- oder Verschließanlagen üblicher Leistung können allein die eingesparten Betriebskosten in der Größenordnung von mehreren hundert tausend Euro liegen. Trotz der verkürzten Behandlungszeit besteht die Möglichkeit, in der wenigstens einen zur Aktivierung des Sterilisationsmediums dienenden Behandlungsstation zugleich auch das Trocknen der Kappen 2 zu erreichen. Weiterhin wird trotz der verkürzten Behandlungszeit während der Aktivierungsphase erreicht, dass die Restmenge an Wasserstoffperoxid in dem aus der betreffenden Behandlungsstation abgeführten Behandlungsmedium deutlich unter einem gesetzlich vorgeschriebenen Wert liegt. In jedem Fall sind aber die Verfahrensschritte bzw. Behandlungsphasen so gesteuert, dass in diesen Behandlungsphasen ein definiertes Temperaturprofil eingehalten wird, um einerseits die Forderungen hinsichtlich eines optimalen Sterilisationseffektes und einer möglichst geringen Wasserstoffperoxid-Festmenge zu erfüllen, und andererseits sicherzustellen, dass die Oberflächentemperatur der Kappen 2 einen oberen Grenzwert, beispielsweise eine Oberflächentemperatur von 70°C nicht übersteigt, um so eine thermische Schädigung der Kappen 2, insbesondere auch solche aus Kunststoff, bzw. eine Beeinträchtigung der Festigkeit und mechanische Stabilität der Kappen 2 zu vermeiden.

Zum Zuführen der Behandlungsmedien an die einzelnen Behandlungsstationen 11 sind Verteilerrohre 17 - 20 mit entsprechenden Anschlüssen 17.1 - 20.1 zum Anschließen an äußere Versorgungsleitungen vorgesehen. Die Verteilerrohre 17 und 18 dienen dabei zum Zuführen des Wasserstoffperoxid enthaltenden Sterilisationsmediums, und zwar der Verteilerkanal 17 an die bezogen auf die Schlaufe des Transportbandes 4 außen liegenden Behandlungsstationen 11 und der Verteilerkanal 18 an die bezogen auf die Schlaufe des Transportbandes 4 innen liegenden Behandlungsstationen 11. Die Verteilerrohre 19 und 20 dienen zum Zuführen des sterilen dampf- oder gasförmigen Mediums, beispielsweise zum Zuführen von steriler Luft, und zwar der Verteilerkanal 19 an die außen liegenden Behandlungsstationen 11 und der Verteilerkanal 20 an die innen liegenden Behandlungsstationen 11. Weiterhin ist im Bereich der Sterilisationstrommel 9 wenigstens ein Abluftkanal vorgesehen.

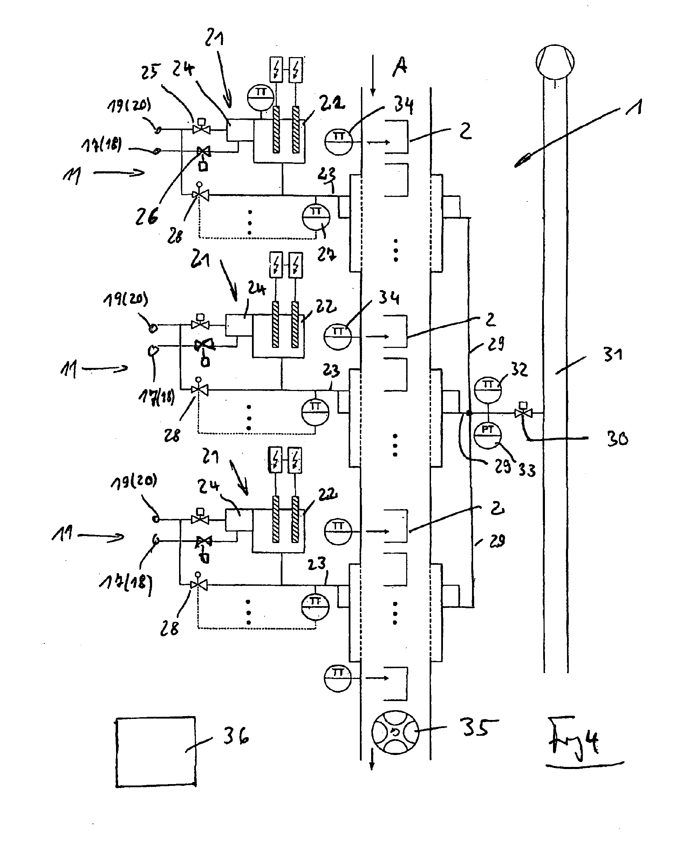

Die Fig. 4 zeigt nochmals in vereinfachter Darstellung die einzelnen in Transportrichtung A aufeinander folgenden Behandlungsstationen 11, wobei in dieser Figur lediglich eine Gruppe der Behandlungsstationen 11, nämlich beispielsweise die außen liegenden Behandlungsstationen 11 oder die innen liegenden Behandlungsstationen 11 dargestellt sind.

Jede Behandlungsstation 11 umfasst bei der dargestellten Ausführungsform in sehr kompakter Bauweise eine Einrichtung 21 zum wahlweisen Bereitstellen von erhitzter steriler Luft zum Aktivieren des Sterilisationsmediums sowie auch zum Bereitstellen des heißen Sterilisationsmediums in Form des Gemisches aus erhitzter Luft und Wasserstoffperoxid (H2O2). Hierfür ist die Einrichtung 21 jeder Behandlungsstation 11 u.a. mit einem Erhitzer 22 ausgebildet, der ausgangsseitig über eine Leitung 21 mit Austrittsdüsen oder Öffnungen der betreffenden Behandlungsstation 11 verbunden ist. Eingangsseitig ist dem Erhitzer 22 ein Mischkopf 24 zugeordnet bzw. vorgeschaltet, der seinerseits über ein Steuerventil 25 mit dem Verteilerkanal 19 bzw. 20 zum Zuführen der sterilen Luft verbunden ist. Gleichzeitig ist der Mischkopf 24 über ein Steuerventil 26 mit dem Wasserstoffperoxid führenden Verteilerkanal 17 bzw. 18 verbunden, sodass durch entsprechende Ansteuerung der Steuerventile 25 und 26 im Mischkopf 24 durch fein verteiltes Einsprühen von Wasserstoffperoxid in den Sterilluftstrom ein Aerosol aus steriler Luft und Wasserstoffperoxid erzeugt werden kann, welches (Aerosol) dann zur Bildung des heißen Sterilisationsmediums im Erhitzer 22 erhitzt wird.

Über ein von einem Temperatursensor 27 in der Leitung 23 gesteuertes Steuerventil 28 ist die Leitung 23 mit dem Verteilerkanal 19 bzw. 20 verbunden. Die Behandlungsstationen 11 sind weiterhin über eine Abluftleitung 29 mit Steuerventil 30 an einen Abluftkanal 31 angeschlossen. In der für sämtliche Behandlungsstationen gemeinsamen Abluftleitung 29 sind u.a. ein Temperatursensor 32 sowie auch ein Sensor 33 vorgesehen, der beispielsweise als Druck- oder Durchflussmesser und/oder als Sensor zur Erfassung des H2O2-Anteils in der über die Abluftleitung 29 abgeführten Abluft ausgebildet ist. Jeder Behandlungsstation 11 ist beispielsweise weiterhin ein zusätzlicher, die Temperatur der von der Behandlungsstation 11 gebildeten Behandlungszone und/oder der behandelten Kappen 2 erfassender Sensor 34 zugeordnet. Am Ausgang der von den Behandlungsstationen 11 gebildeten Behandlungsstrecke ist beispielsweise eine Sensoreinrichtung 35, z.B. in Form eines Kappenrades vorgesehen, die (Sensoreinrichtung) mit einer Steuerelektronik 36 zusammenwirkt und mit der die Anzahl der die Behandlungsstationen 11 bzw. die Behandlungsstrecke tatsächlich passierenden Kappen 2 und damit die tatsächliche Leistung der Vorrichtung 1 erfasst werden.

Eine Besonderheit der von den Behandlungsstationen 11 gebildeten Behandlungsstrecke bzw. der Vorrichtung 1 besteht darin, dass jede Behandlungsstation 11 als Applikationsstation zum Applizieren des Wasserstoffperoxid enthaltenden heißen Sterilisationsmediums betrieben werden kann, und zwar dadurch, dass die entsprechende Einrichtung 21 an der Leitung 23 das heiße Sterilisationsmedium bereitstellt, wofür über das geöffnete Steuerventil 26 Wasserstoffperoxid im Mischkopf 24 in den über das geöffnete Steuerventil 25 zugeführten Sterilluftstrom eingesprüht wird, sodass dieses Gemisch bzw. Aerosol dann nach dem Erhitzen im Erhitzer 22 an der Leitung 23 als heißes Sterilisationsmedium bereitsteht und auf die an der betreffenden Behandlungsstation 11 getaktet vorbeibewegten Kappen 2 aufgebracht werden kann.

Durch entsprechende Ansteuerung, insbesondere der Steuerventile 25, 26 und 28 kann jede Behandlungsstation 11 auch als Aktivierungsstation zum Aktivieren des auf die Kappen 2 aufgebrachten Sterilisationsmedium betrieben werden, und zwar dadurch, dass die entsprechende Einrichtung 21 an ihrer Leitung 23 erhitzte sterile Luft für die Aktivierung bereitstellt, wofür die über das geöffnete Steuerventil 25 zugeführte sterile Luft im Erhitzer 22 erhitzt und zur Temperaturregelung über das Steuerventil 28 nicht erhitzte sterile Luft beigemischt wird.

Weiterhin besteht auch die Möglichkeit, wenigstens eine Behandlungsstation 11 für das Temperieren oder für ein zusätzliches Temperieren der Kappen 2 zu nutzen, und zwar wiederum durch Bereitstellung von erhitzter steriler Luft an der Leitung 23 bzw. an den mit dieser Leitung verbundenen Düsenöffnungen, und zwar durch Erhitzen der Luft im Erhitzer 23 und durch eine Temperaturregelung über das Steuerventil 28 durch Beimischung von nicht erhitzter steriler Luft.

Im Speziellen erfolgt die Steuerung der einzelnen Behandlungsstationen 11 bzw. deren Einrichtungen 21 bevorzugt in Abhängigkeit von der Leistung der Vorrichtung 1 und/oder in Abhängigkeit von der Förder- oder Transportgeschwindigkeit, mit der die Kappen 2 bzw. die Kappenkörbe 4.1 durch die Behandlungsstationen 11 und damit durch deren Behandlungszonen bewegt werden, d.h. in Abhängigkeit von der Transportgeschwindigkeit des Transportbandes 4, und zwar gesteuert durch die Steuereinrichtung 36 derart, dass die Anzahl der jeweils als Applikationsstation und als Aktivierungsstation betriebenen Behandlungsstationen 11 an die Leistung der Vorrichtung und/oder an die Transportgeschwindigkeit des Transportbandes 4 angepasst ist. Bei sehr niedriger Leistung oder Transportgeschwindigkeit wird für die Applikation des Sterilisationsmediums und für die Aktivierung des Sterilisationsmediums beispielsweise jeweils nur eine Behandlungsstation 11 verwendet, während die übrigen Behandlungsstationen deaktiviert sind. Bei höheren Leistungen werden entsprechend mehrere, in Transportrichtung A aneinander anschließende Behandlungsstationen 11 jeweils für die Applikation des Sterilisationsmediums und für die Aktivierung des Sterilisationsmediums verwendet, sodass trotzt der erhöhten Transportgeschwindigkeit des Transportbandes 4 und damit trotz der erhöhten Geschwindigkeit, mit der die Kappen 2 durch die von den Behandlungsstationen 11 gebildete Behandlungsstrecke bewegt werden, der angestrebte Sterilisationseffekt erreicht wird. Erfolgt im Bereich der Sterilisationstrommel 9 auch ein Temperieren, beispielsweise ein Vorwärmen der Kappen 2, so ist es dann auch möglich, die Anzahl der für dieses Temperieren verwendeten Behandlungsstationen 11 in Abhängigkeit von der Transportgeschwindigkeit des Transportbandes 4 und/oder in Abhängigkeit von der tatsächlichen Leistung der Vorrichtung 1 zu steuern oder zu regeln, d.h. bei geringer Leistung und/oder Transportgeschwindigkeit nur eine Behandlungsstation 11 für das Temperieren und bei höherer Leistung oder Transportgeschwindigkeit eine entsprechend höhere Anzahl von Behandlungsstationen 11 für das Temperieren zu nutzen. Durch das Zu- und Wegschalten von Behandlungsstationen 11, welches vorzugsweise jeweils beginnend vom Einlauf 9.1 der Sterilisationstrommel 9 in oder entgegen der Förderrichtung A erfolgt, ist insbesondere auch ein schnelles Anpassen an eine sich ändernde Leistung der Vorrichtung 1 möglich.

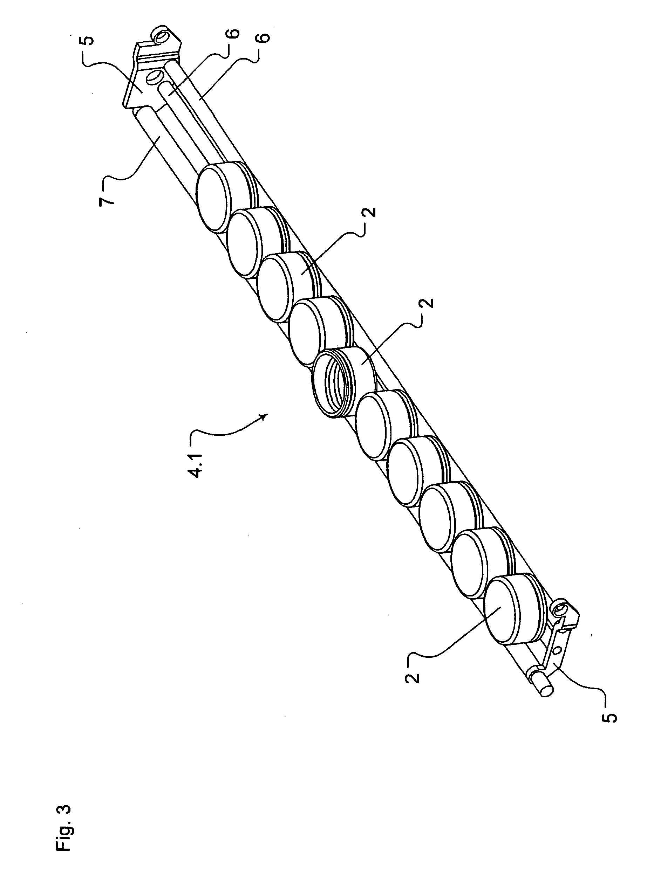

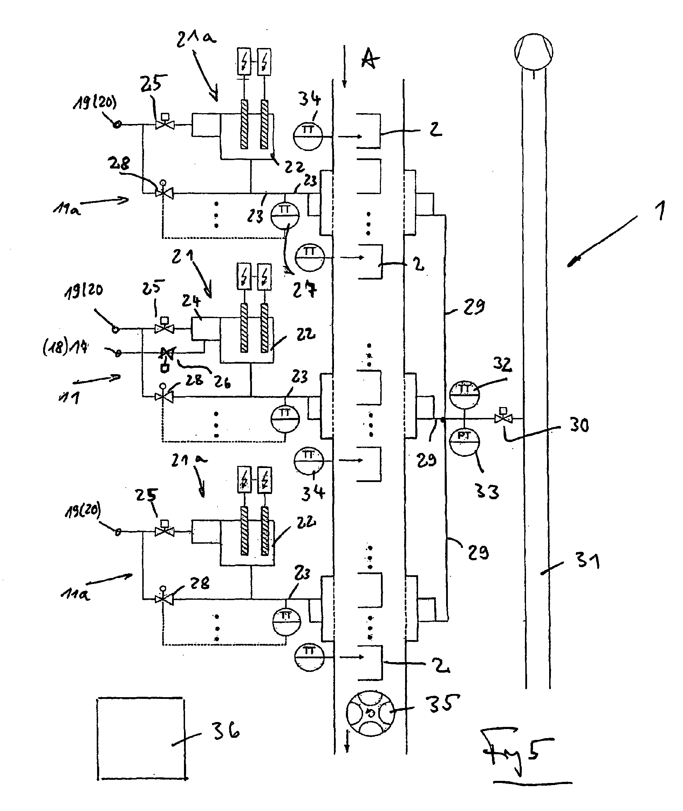

Die Figur 5 zeigt als weitere Ausführungsform eine von der Vielzahl der Behandlungsstationen 11 gebildete Behandlungsstrecke, die sich von der Behandlungsstrecke der Figur 4 im Wesentlichen dadurch unterscheidet, dass in Transportrichtung A aufeinander folgend mehrere Gruppen, beispielsweise drei Gruppen von Behandlungsköpfen 11a und 11 vorgesehen sind, und zwar in Transportrichtung auf den Einlauf 9.1 folgend eine Gruppe von Behandlungsstationen 11a, daran anschließend eine Gruppe von Behandlungsstationen 11 und daran anschließend wieder eine Gruppe von Behandlungsstationen 11a. Der einfacheren Darstellung wegen ist in der Figur 3 für jede Gruppe allerdings nur eine Behandlungsstation 11 bzw. 11a gezeigt.

Den Behandlungsstationen 11 ist wiederum jeweils eine Einrichtung 21 zugeordnet und den Behandlungsstationen 11a jeweils eine Einrichtung 21a, die sich von der Einrichtung 21 im Wesentlichen dadurch unterscheidet, dass die Mittel zum Zumischen oder Einbringen von Wasserstoffperoxid in den Sterilluftstrom (z.B. Mischkopf 24, Ventil 26 und der Anschluss zum Verteilerkanal 17 oder 18) fehlen, die Behandlungsköpfe 11a also jeweils ausschließlich als Vorbehandlungskopf für das Temperieren oder als Aktivierungskopf zum Aktivieren des Sterilisationsmediums betrieben werden können.

Die Steuerung der einzelnen Behandlungsköpfe 11 und 11 a bzw. deren Einrichtungen 21 oder 21a erfolgt dann beispielsweise generell so,
dass die Kappen 2 wiederum nach dem Erreichen der Sterilisationstrommel 9 beispielsweise zusätzlich in einem oder aber in mehreren Verfahrensschritten temperiert oder vorgewärmt werden, wofür wenigstens eine Behandlungsstation 11 a der ersten Gruppe als Vorwärm- oder Vorbehandlungsstation betrieben wird,
dass in einem weiteren Verfahrensschritt das Beaufschlagen der Kappen 2 mit dem heißen Sterilisationsmedium bestehend aus der heißen sterilen Luft und dem Wasserstoffperoxid erfolgt, wofür wenigstens eine Behandlungsstation 11 der zweiten Gruppe als Applikationsstation betrieben wird, und
dass in einem weiteren Verfahrensschritt das Aktivieren des Sterilisationsmediums erfolgt, wofür wenigstens eine Behandlungsstation der dritten Gruppe als Aktivierungsstation betrieben wird.

In Abhängigkeit von der Leistung der Vorrichtung 1 und/oder in Abhängigkeit von der Transportgeschwindigkeit des Transportbandes 4 erfolgt auch bei dieser Ausführung ein gesteuertes Zu- und Wegschalten der aktivierten Behandlungsstationen 11 und 11a, und zwar dann innerhalb der jeweiligen Gruppe. Ist ein Temperieren oder Nachtemperieren der Kappen 2 an der Sterilisationstrommel 9 nicht erforderlich, so sind dort abweichend von der Darstellung der Figur 5 nur zwei Gruppen von Behandlungsstationen erforderlich, nämlich die Behandlungsstationen 11 als erste Gruppe und die Behandlungsstationen 11a als in Transportrichtung A anschließende zweite Gruppe.

Wie vorstehend erwähnt, zeigen die Figuren 4 und 5 in vereinfachter Darstellung jeweils nur die Behandlungsstationen 11 und 11a, die im Bereich der Sterilisationstrommel 9 an einer Seite des Transportelementes 4 vorgesehen sind, beispielsweise an der bezogen auf die Schlaufe des Transportelementes 4 außen liegenden Seite. An der innen liegenden Seite sind dann die dortigen Behandlungsstationen 11 oder 11a in gleicher Weise ausgeführt.

Vorstehend wurde davon ausgegangen, dass jede Behandlungsstation 11 und 11a u.a. einen eigenständigen Erhitzer 22 sowie jede Behandlungsstation 11 auch einen eigenständigen Mischkopf 24 aufweist und weiterhin die Steuerventile 25, 26 und 28 sowie Temperatursensoren 27 für die Behandlungsstationen jeweils eigenständig vorgesehen sind. Selbstverständlich besteht die Möglichkeit, zur Vereinfachung der Behandlungsstationen 11 und 11a die vorgenannten Elemente, soweit erforderlich, für sämtliche Behandlungsstationen 11 oder 11a oder aber für Gruppen solcher Behandlungsstationen gemeinsam vorzusehen, beispielsweise auch in der Form, dass die vorgenannten Funktionselemente oder Einrichtungen 10 bzw. 10a für einander zugeordnete außen liegende und innen liegende Behandlungsstationen 11 und 11a oder aber für eine Gruppe solcher Behandlungsstationen gemeinsam vorgesehen sind.

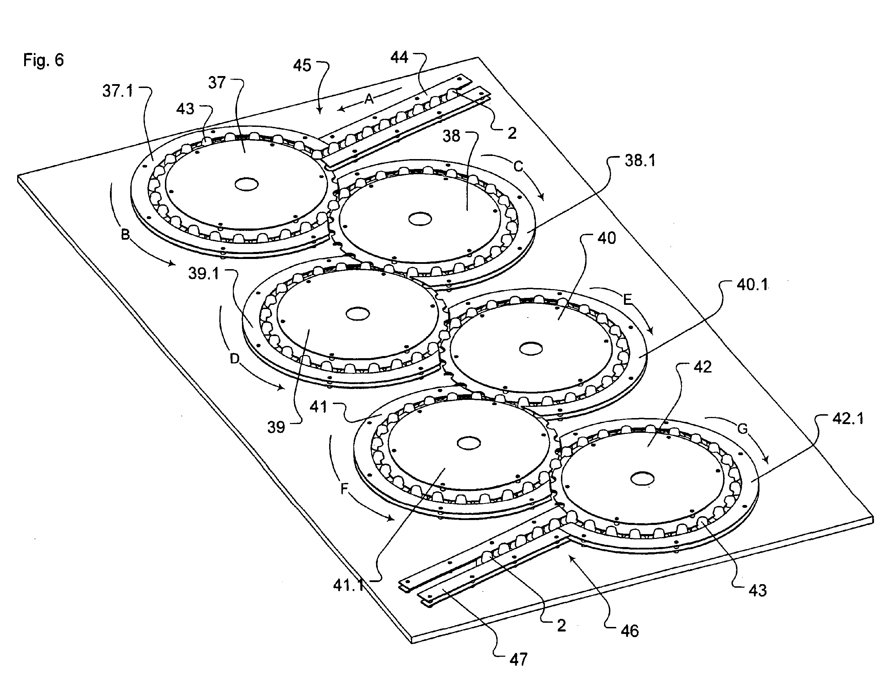

Die Figur 6 zeigt in sehr schematischer Darstellung eine Vorrichtung, 1a nicht gemäß der Erfindung bzw. deren Transporteur 3a, der von mehreren in Transportrichtung A unmittelbar aneinander anschließenden Transporträdern 37 - 42 mit zugehörigen äußeren Führungen 37.1 - 42.1 gebildet ist. Die Transporträder 37 - 42 sind an ihrem Umfang jeweils mit einer Vielzahl von Aufnahmen 43 zur Aufnahme wenigstens einer Kappe 2 ausgebildet und um parallele Achsen gegenläufig (Pfeile B - G), aber synchron angetrieben, und zwar derart, dass jede über eine Zuführung 44 einem Kappeneinlass 45 der Vorrichtung 1a zugeführte Kappe 2 durch Weitergabe von einem Transportrad 37 - 41 an das in Transportrichtung A jeweils anschließende Transportrad 38 - 42 an den Kappenauslass 46 gelangt, an den sich ein an die Verschließstation einer Verschließmaschine führender Kappenkanal 47 anschließt.

Die in den Aufnahmen 43 aufgenommenen Kappen 2 werden somit durch die gegenläufig umlaufend angetriebenen Transporträder 37 - 42 auf von diesen gebildeten und aneinander anschließenden kreisbogenförmigen Teilstrecken der Behandlungs- oder Transportstrecke des Transporteurs 3a bewegt, und zwar vorbei an nicht dargestellten den Behandlungsstationen 11 oder 11 und 11a entsprechenden Behandlungsstationen, an denen das Sterilisieren der Kappen 2 erfolgt, beispielsweise durch vorausgehendes Temperieren oder Vorwärmen, durch anschließendes Beaufschlagen der Kappen 2 mit dem Wasserstoffperoxid enthaltenden Sterilisationsmedium und durch anschließendes Aktivieren des Sterilisationsmediums durch Beaufschlagen der Kappen 2 mit dem heißen dampf- und/oder gasförmigen Aktivierungsmedium, beispielsweise durch Beaufschlagung mit heißer steriler Luft.

Bevorzugt werden die einzelnen Verfahrensschritte jeweils an getrennten Transporträdern 37 - 42 durchgeführt, d.h. die für die unterschiedlichen Verfahrensschritte erforderlichen Behandlungsstationen sind jeweils an eigenständigen Transporträdern 37 - 42 vorgesehen. Der Vorteil der Vorrichtung 1 a gegenüber der Vorrichtung 1 besteht u.a. darin, dass die Transporträder 37 - 42 und deren Aufnahmen 43 wesentlich einfacher und schneller zusammen mit den Kappen 2 sterilisiert werden können, als dies bei dem gliederkettenartigen Transportband 4 des Transportelementes 3 möglich ist. Weiterhin kann der Transporteur 3a preisgünstig und sehr kompakt, d.h. mit geringem Bauvolumen realisiert werden. Die einzelnen Transporträder 37 - 42 und die zugehörigen Führungen 37.1 - 42.1 können bei Formatänderungen, d.h. zur Verarbeitung von Kappen 2 oder anderen Verschlüssen unterschiedlicher Größe leicht gewechselt werden.

Die Transporträder 37 - 42 sind beispielsweise so ausgebildet, dass in jeder Aufnahme 43 nur jeweils eine Kappe 2 Platz findet. Grundsätzlich besteht aber auch die Möglichkeit, die Transporträder 37 - 42 trommelartig auszuführen, und zwar derart, dass in jeder Aufnahme 43 in Richtung der Achse des jeweiligen Transportrades wenigstens zwei oder aber auch mehr als zwei Kappen 2 Platz finden.

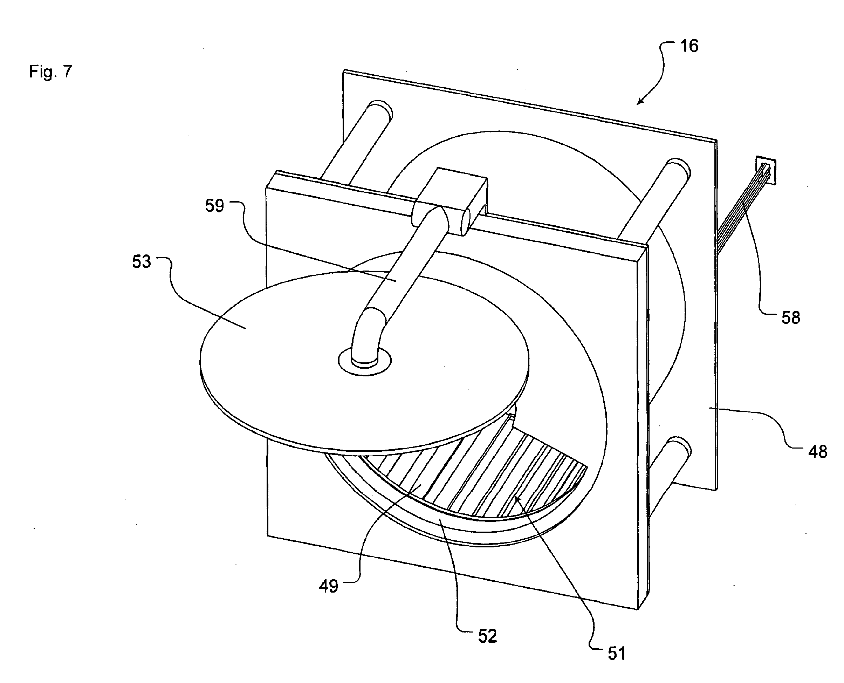

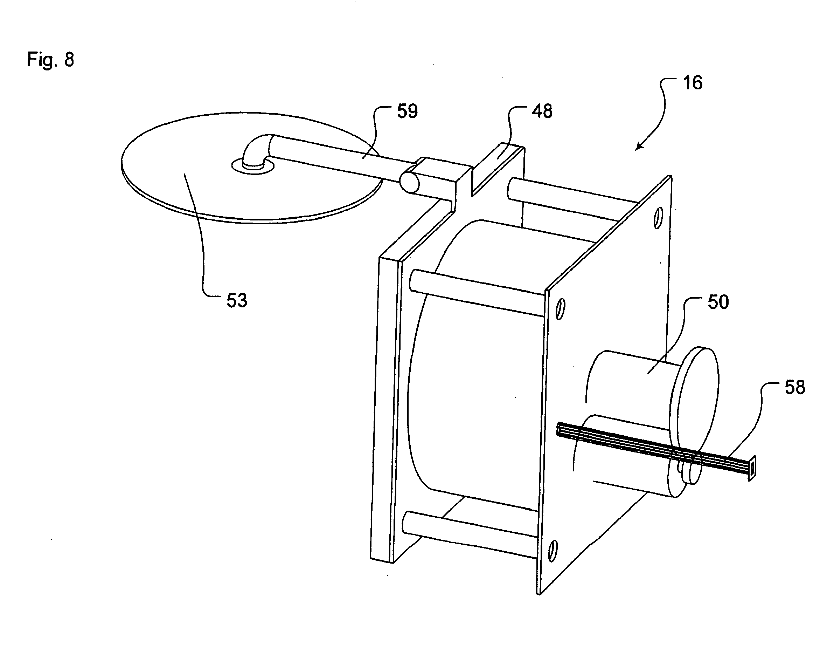

Die Figuren 7 - 9 zeigen als weitere Ausführungsform eine Vorrichtung 1b zum Sterilisieren und/oder Desinfizieren der Kappen 2. Die Vorrichtung 1 b besteht im Wesentlichen aus einem Gehäuse 48, in welchem eine Trommel 49 vorgesehen ist, die um ihre horizontale Trommelachse TA durch einen Antrieb 50 umlaufend angetrieben werden kann (Pfeil F), und zwar vorzugsweise getaktet. Die Trommel 49 ist so ausgeführt, dass sie im Wesentlichen aus einer die Trommelachse TA konzentrisch umschließenden kreiszylinderförmigen Trommelwand 49.1 sowie aus einem Trommelboden 49.2 besteht, an welchem die Trommel 49 mittels einer nicht dargestellten Welle in einem Lager des Gehäuses 48 drehbar gelagert ist. An der Vorderseite der Vorrichtung 1 b bzw. des Gehäuses 48 ist der Innenraum 51 der Trommel 49 über eine bei der dargestellten Ausführungsform kreisförmige und in der vorderen Wand des Gehäuses 48 vorgesehene sowie durch eine kreisscheibenförmige Klappe 53 verschließbare Öffnung 52 zugänglich. Die Klappe 53 besteht beispielsweise aus einem transparenten Material, beispielsweise aus Acrylglas, so dass die Arbeitsweise der Vorrichtung 1 b optisch überwacht werden kann.

An der Innenfläche ist die kreiszylinderförmige Trommelwand 49.1 mit einer Vielzahl von Vorsprüngen oder Stegen 54 versehen, die sich jeweils parallel zur Trommelachse TA über die gesamte axiale Breite der Trommelwand 49.1 erstrecken, so dass jeweils zwischen zwei in Umfangsrichtung der Trommel 49 aufeinander folgenden Stegen 54 eine Aufnahme 55 zur Aufnahme einer Vielzahl von Kappen 2 gebildet sind, die in jeder Aufnahme in einer Reihe parallel zur Trommelachse TA angeordnet sind.

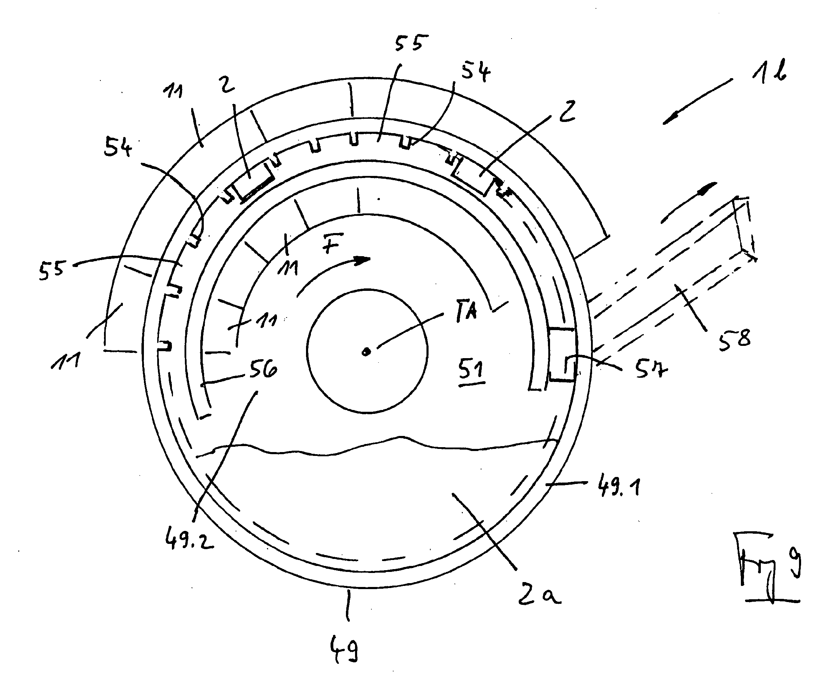

Mit 56 ist in der Figur 9 eine innen liegende Führung bezeichnet, die sich kreisbogenförmig über einen Winkelbereich der Drehbewegung der Trommel 49 erstreckt, aber mit dieser Trommel nicht mitdrehend in geeigneter Weise am Gehäuse 48 gehalten ist. Die Führung 56 ist so angeordnet und ausgebildet, dass die in den Aufnahmen 55 aufgenommenen Kappen 2 zumindest auf dem Winkelbereich der Drehbewegung der Trommel 49 durch die Führung 56 abgestützt sind, auf dem (Winkelbereich) die Kappen 2 ohne die Führung 56 aufgrund der Schwerkraft aus den Aufnahmen 55 herausfallen würden. Die Führung 56 reicht bis an einen Kappenauslass 57, an dem die einzelnen Aufnahmen 55 mit der Trommel 49 vorbei bewegt werden und an dem die in der jeweiligen Aufnahme 55 angeordneten Kappen 2 als Kappengruppe, in der die Kappen 2 in Reihe nebeneinander angeordnet sind, in einem Kappenkanal 58 ausgebracht werden, und zwar zur Weiterleitung beispielsweise an die Kappenaufnahme einer Verschließmaschine.

Die zu behandelnden bzw. zu sterilisierenden und/oder zu desinfizierenden Kappen 2 werden dem Trommelinnenraum 51 über eine Kappenzuführung zugeführt. Bei der dargestellten Ausführung ist diese Zuführung von einem Rohrstück 59 gebildet, welche zugleich auch die Halterung für die schwenkbare Klappe 53 ist. Über das Rohrstück 59 gelangen die zu behandelnden Kappen 2 im Bereich der Trommelachse TA in den Trommelinnenraum 51, und zwar innerhalb der Führung 56 und bilden dann im unteren Bereich des Trommelinnenraumes 51 einen in der Figur 9 mit 2a bezeichneten Kappenvorrat, der die Kappen 2 in ungeordneter Menge enthält.

Durch die Drehbewegung der Trommel 49 werden die Kappen 2 in dem Kappenvorrat 2a über die gesamte axiale Breite der Trommel 49 verteilt und mit den Aufnahmen 55 sortiert mitgeführt, so dass die Kappen 2 in diesen Aufnahmen 55 eine vorgegebene Orientierung aufweisen, und zwar beispielsweise mit der offenen Kappenseite bezogen auf die Trommelachse TA radial nach innen orientiert. Diese Sortierung ist beispielsweise durch entsprechende Ausbildung der Stege 54 erreichbar.

Entlang der Transportstrecke, auf der die in den Aufnahmen 55 angeordneten Kappen bis an den Kappenauslass 58 bewegt werden und entlang der sich auch die Führung 56 erstreckt, sind mehrere Behandlungsköpfe oder -stationen 11 vorgesehen, die sich jeweils über die gesamte axiale Breite der Trommel 49 erstrecken und mit denen zumindest das Sterilisieren und/oder Desinfizieren der Kappen 2 erfolgt. Die Behandlungsstationen 11 sind dabei so angeordnet und/oder ausgebildet, dass eine Behandlung der Kappen 2 an ihrer gesamten Innen- und Außenfläche erfolgt, beispielsweise zumindest durch Aufbringen des heißen Wasserstoffperoxids enthaltenden Sterilisationsmediums in einer Applikationsphase und durch anschließendes Aktivieren dieses Sterilisationsmediums mit dem heißen Aktivierungsmedium.

Auch bei dieser Ausführungsform ist es wiederum möglich, die Dreh- oder Fördergeschwindigkeit der Trommel 49 und damit die Geschwindigkeit, mit der die Kappen 2 durch die Behandlungszonen 11 bewegt werden, in Abhängigkeit vom Bedarf an behandelten Kappen 2, d.h. in Abhängigkeit von der Leistung der Verschließmaschine zu steuern, wobei die Anzahl der jeweils aktivierten Behandlungsköpfe oder -stationen 11 ebenfalls in Abhängigkeit von der Fördergeschwindigkeit oder der Leistung gesteuert wird, so dass die erforderlichen Behandlungszeiten in optimaler Weise eingehalten werden.

Das Ausbringen der behandelten Kappen 2 an dem Kappenauslass 57 in den Kappenkanal 58 erfolgt beispielsweise durch einen mechanischen Ausstoßer oder Schieber, der synchron mit der Drehbewegung der Trommel 49 betätigt wird und/oder durch Blasen mit einem sterilen gas- und/oder dampfförmigen Medium, beispielsweise mit steriler Blasluft.

Der besondere Vorteil der Vorrichtung 1 b besteht u.a. in einer vereinfachten konstruktiven Ausbildung sowie insbesondere auch darin, dass mit der Trommel 49 sowohl die Sortierung der Kappen 2, als auch die Bewegung der Kappen 2 durch die von den Behandlungsstationen 11 gebildete Behandlungsstrecke erfolgt. Weiterhin ist die Vorrichtung 1 für eine kontinuierliche Behandlung der Kappen 2 geeignet, und zwar dadurch, dass die zu behandelnden Kappen 2 dem Trommelinnenraum 51 über das Rohrstück 59 gesteuert zugeführt und mit der umlaufenden Trommel 49 kontinuierlich aus dem Kappenvorrat 2a entnommen werden. Ein Öffnen der Klappe 53 ist dabei nur im Falle einer Störung erforderlich.

Die Vorrichtung 1 b eignet sich aber auch für eine chargenweise Behandlung der Kappen 2, beispielsweise in der Form, dass jeweils eine Anzahl von Kappen 2 über die geöffnete Klappe 43 in den Trommelinnenraum 51 eingebracht, die Kappen 2 dann in der vorbeschriebenen Weise sortiert mit den Aufnahmen 55 an den Behandlungsstationen 11 behandelt und die sterilisierten und/oder desinfizierten Kappen 2 über den Kappenauslass 57 den Kappenkanal 58 zugeführt werden.

Ein besonderer Vorteil der Vorrichtung 1b besteht weiterhin auch darin, dass die Trommel 59 insbesondere an ihrer mit den Kappen 2 in Berührung kommenden Trommelinnenfläche zumindest weitestgehend glatt und ohne schwer zugängliche Hinterschneidungen, Zwischenräume, Spalten usw. ausgebildet ist und die Trommel 49 und deren Aufnahmen 55 nicht nur beim Vorbeibewegen an den Behandlungsstationen 11 ebenfalls ständig sterilisiert und/oder desinfiziert werden, sondern sich auch außerhalb der von den Behandlungsstationen 11 gebildeten Behandlungsstrecke in einem sterilen Raum oder einem weitestgehend sterilen Raum bewegen, so dass bei vereinfachter konstruktiver Ausbildung und hoher Betriebssicherheit der Vorrichtung 1 b mit dieser Vorrichtung auch eine hohe Qualität der Sterilisation bzw. eine hohe Entkeimungsrate erreichbar sind.

Die Erfindung wurde voranstehend an Ausführungsbeispielen beschrieben. Es versteht sich, dass zahlreiche Änderungen sowie Abwandlungen möglich sind, ohne dass dadurch der der Erfindung zugrunde liegende Erfindungsgedanke verlassen wird. So eignen sich die Vorrichtungen 1 und 1 a beispielsweise zum Sterilisieren nicht nur von Kappen 2 im eigentlichen Sinne, sondern auch zum Sterilisieren von Verschlüssen anderer Art, die zum Verschließen von Flaschen oder dergleichen Behältern verwendet werden. Weiterhin besteht die Möglichkeit, sämtliche Behandlungsstationen an einem gemeinsamen Transportrad oder an einer gemeinsamen Transporttrommel vorzusehen.

Allen beschriebenen Ausführungsformen der Erfindung ist aber gemeinsam, dass das jeweilige Transportelement oder der jeweilige Transporteur Körbe oder Aufnahmen für die Kappen 2 bildet, in denen die Kappen 2 bei ihrem Transport zwischen einer Verschlussaufgabe, beispielsweise dem Bunker 8 oder dem Kappeneinlass 45 oder dem Kappenvorrat 2a und einer Verschlussabgabe, beispielsweise dem Kappenauslass 12 oder 46 oder 57 am Transportelement oder Transporteur zumindest in Transportrichtung A oder F definiert und/oder formschlüssig aufgenommen sind, sodass eine insbesondere auch in Abhängigkeit von der Leistung der Vorrichtung 1 bzw. 1 a oder der Transportgeschwindigkeit optimal gesteuerte Behandlung der Kappen 2 möglich ist, und zwar durch Einhaltung der für eine optimale Sterilisation erforderlichen Behandlungszeiten an den einzelnen Behandlungsstationen, sowie auch ein vereinfachtes Einbringen der Kappen 2 am Kappenauslass 12 oder 46 oder 57 in einen dort anschließenden und an die Verschließstation einer Verschließmaschine führenden Kappenkanal 14 oder 47 oder 58.

### Bezugszeichenliste

- 1, 1a: Vorrichtung
- 2: Verschluss oder Kappe
- 2a: Kappenvorrat
- 3: Transporteur oder Transportelement
- 3.1: Transportstrecke
- 3.2: Leerstrecke
- 4: Transportband
- 4.1: Kappenkorb
- 5: Endstück
- 6, 7: Steg
- 8: Bunker
- 9: Sterilisationstrommel
- 9.1: Einlauf der Sterilisationstrommel bzw. der Behandlungsstrecke
- 10: Antriebsmotor
- 11, 11a: Behandlungsstation
- 12: Kappenauslass
- 13: Ausstoßeinrichtung
- 14: Kappenkanal
- 15: Kappenzuführung an Bunker 8
- 16: Sortierstation
- 17 - 20: Verteilerrohr
- 17.1 - 20.1: Anschluss
- 21: Einrichtung
- 22: Erhitzer
- 23: Leitung
- 24: Mischkopf
- 25, 26: Steuerventil
- 27: Temperatursensor
- 28: Steuerventil
- 29: Leitung
- 30: Ventil
- 31: Abluftkanal
- 32, 33, 34: Sensor
- 35: Sensorelement
- 36: Steuereinrichtung
- 37 - 42: Transportrad oder Transporttrommel
- 37.1 - 42.1: Führung
- 43: Aufnahme
- 44: Zuführung
- 45: Kappeneinlass
- 46: Kappenauslass
- 47: Kappenkanal
- 48: Gehäuse
- 49: Trommel
- 49.1: Trommelumfangswand
- 49.2: Trommelboden
- 50: Antrieb
- 51: Trommelinnenraum
- 52: Öffnung an der Vorderseite des Gehäuse 48
- 53: Klappe zum Verschließen der Öffnung 52
- 54: Steg
- 55: Aufnahme
- 56: Führung
- 57: Kappenauslass
- 58: Kappenkanal
- 59: Rohrstück

- A: Transportrichtung
- B - G: Drehrichtung der Transporträder 37 - 42
- F: Drehrichtung der Trommel 59
- TA: Trommelachse

## Patentansprüche

1. Vorrichtung zum Sterilisieren von Kappen oder dergleichen Verschlüssen (2) zum Verschließen von Flaschen oder dergleichen Behältern, mit zumindest einer Behandlungsstation zum Sterilisieren der Verschlüsse (2) und mit einem die Verschlüsse (2) zumindest auf einer Behandlungsstrecke und/oder in einem Behandlungsraum zum Sterilisieren der Verschlüsse (2) bewegenden umlaufenden Transportelement (4; 37 - 42; 49), wobei das Transportelement (4; 37-42; 49) in einer Transport- oder Umlaufrichtung (A, F) aufeinander folgend mehrere Aufnahmen (43, 55) oder Körbe (4.1) zur Aufnahme jeweils wenigstens eines Verschlusses (2) in der Form aufweist, dass die in den Aufnahmen (43, 55) oder Körben (4.1) angeordneten Verschlüsse (2) zumindest In Transport- oder Bewegungsrichtung (A, F) eine definierte Lage und/oder Orientierung in Bezug auf das Transportelement (4; 37 - 42; 49) aufweisen,
**dadurch gekennzeichnet, dass**
das Transportelement (4, 49) für die Aufnahme der Verschlüsse (2) aus einer ungeordneten Menge und zum Sortieren der Verschlüsse (2) ausgebildet ist, so dass die Verschlüsse die definierte Lage und/oder Orientierung aufweisen.

2. Vorrichtung nach Anspruch 1, **dadurch gekennzeichnet, dass** das wenigstens eine umlaufende Transportelement (4; 37 - 42; 49) Bestandteil eines Transportsystems (3, 3a) ist, mit dem die Verschlüsse (2) in einer Transportrichtung (A, F) zumindest durch eine Behandlungsstrecke mit wenigstens einer Behandlungsstation zum Sterilisieren der Verschlüsse, vorzugsweise durch eine Behandlungsstrecke mit mehreren in Transportrichtung (A, F) aufeinander folgenden Behandlungsstationen (11, 11 a) bewegt werden.

3. Vorrichtung nach einem der vorhergehenden Ansprüche, **dadurch gekennzeichnet, dass** die Verschlussaufnahmen oder Körbe (43, 4.1) jeweils zur Aufnahme von mehreren quer oder senkrecht zur Transportrichtung (A) aneinander anschließenden oder gegeneinander versetzten Verschlüssen (2) ausgebildet sind.

4. Vorrichtung nach einem der vorhergehenden Ansprüche, **dadurch gekennzeichnet, dass** das Transportelement ein eine geschlossene Schlaufe bildendes und endlos umlaufend antreibbares Transportband (4) ist, und dass am Transportband (4) in Transportrichtung (A) aufeinander folgend eine Vielzahl von Verschlussaufnahmen oder Körben (4.1) vorgesehen ist.

5. Vorrichtung nach Anspruch 4, **dadurch gekennzeichnet, dass** das Transportband (4) gliederkettenartig mit mehreren in Transportrichtung (A) aneinander anschließenden und gelenkig miteinander verbundenen Behalterkörben (4.1) ausgebildet ist.

6. Vorrichtung nach Anspruch 4 oder 5, **dadurch gekennzeichnet, dass** die Körbe (4.1) jeweils käfigartig für eine möglichst frei liegende Aufnahme der Verschlüsse (2) ausgebildet sind.

7. Vorrichtung nach einem der Ansprüche 4 bis 6, **dadurch gekennzeichnet, dass** das Transportband (4) durch einen Bunker (8) zur Aufnahme der Verschlüsse (2) geführt ist, und zwar bevorzugt im Bereich einer Umlenkung des Transportbandes (4).

8. Vorrichtung nach einem der Ansprüche 4 bis 7, **dadurch gekennzeichnet, dass** an der Bewegungsbahn der Körbe (4.1) oder Aufnahmen (43) Mittel vorgesehen sind, beispielsweise in Form von mechanischen Schikanen und/oder Führungen und/oder von gesteuerten Blasdüsen, mit denen falsch orientierte Verschlüsse (2) aus den Körben (4.1) oder Aufnahmen (43) entfernt werden, bevor solche Verschlüsse (2) die Behandlungsstationen (11, 11a) oder die von diesen gebildete Behandlungsstrecke erreichen.

9. Vorrichtung nach einem der Ansprüche 4 bis 8, **dadurch gekennzeichnet, dass** die Behandlungsstationen (11, 11a) in Transportrichtung (A) aufeinander folgend an wenigstens einer Seite einer vom Transportband (4) gebildeten Schlaufe, vorzugsweise an beiden Seiten dieser Schlaufe vorgesehen sind, und zwar bevorzugt im Bereich einer Umlenkung des Transportbandes bzw. im Bereich einer diese Umlenkung bildenden Sterilisationstrommel (9).

10. Vorrichtung nach einem der vorhergehenden Ansprüche, **dadurch gekennzeichnet, dass** an der Behandlungsstrecke oder an den Behandlungsstationen (11, 11a) Mittel vorgesehen sind, um die Verschlüsse (2) In dem jeweiligen Korb (4.1) oder in der jeweiligen Aufnahmen (43) quer oder senkrecht zur Transportrichtung (A) zu bewegen und/oder von einander zu beabstanden.

11. Vorrichtung nach einem der Ansprüche 7 bis 10, **dadurch gekennzeichnet, dass** der Bunker (8) mit einer Heizeinrichtung zum Vorwärmen der Verschlüsse (2) ausgebildet ist.

12. Vorrichtung nach einem der vorhergehenden Ansprüche, **dadurch gekennzeichnet, dass** an einem Verschlussauslass (12) eine Ausstoßeinrichtung (13) vorgesehen ist, und zwar zum zeitgleichen Ausbringen von jeweils mehreren in wenigstens einem Korb (4.1) oder einer Aufnahme (43) angeordneten Verschlüssen (2) vorzugsweise in einen an den Verschlussauslass (12, 46) anschließenden Kappenkanal (14, 47).

13. Vorrichtung nach einem der vorhergehenden Ansprüche, **dadurch gekennzeichnet, dass** wenigstens eine Behandlungsstation (11) zum Aufbringen eines vorzugsweise heißen, Wasserstoffperoxid enthaltenden Sterilisationsmediums auf die Verschlüsse (2) und wenigstens eine in Transportrichtung (A) folgende Behandlungsstation zum Aktivieren des Wasserstoffperoxids durch Erhitzen, beispielsweise zum Ausbringen eines erhitzten dampf- und/oder gasförmigen Mediums, beispielsweise zum Ausbringen von erhitzter, steriler Luft und/oder zur Beaufschlagung der Verschlüsse (2) mit Strahlungsenergie, beispielsweise mit Infrarot-Strahlung ausgebildet sind.

14. Vorrichtung nach Anspruch 13, **dadurch gekennzeichnet, dass** der wenigstens einen Behandlungsstation zum Beaufschlagen der Verschlüsse (2) mit dem Sterilisationsmedium in Transportrichtung (A) vorausgehend wenigstens eine weitere Behandlungsstation (11, 11a) zum Vorwärmen der Verschlüsse (2), beispielsweise durch Beaufschlagung mit einem erhitzten dampf- und/oder gasförmigen Medium, beispielsweise mit erhitzter Luft oder erhitzter steriler Luft und/oder durch Beaufschlagung mit einer Strahlungsenergie, beispielsweise Infrarot-Strahlung vorgesehen ist.

15. Vorrichtung nach einem der vorhergehenden Ansprüche, **dadurch gekennzeichnet, dass** jeder Behandlungsstation (11) oder aber einer Gruppe von Behandlungsstationen (11, 11a) oder aber sämtlichen Behandlungsstationen (11, 11 a) der Vorrichtung (1, 1 a) ein Erhitzer (22) zugeordnet ist.

16. Vorrichtung nach einem der vorhergehenden Ansprüche, **dadurch gekennzeichnet, dass** zumindest den zum Ausbringen des Sterilisationsmediums dienenden Behandlungsstationen (11) oder einer Gruppe solcher Stationen ein Mischkopf (24) zum Erzeugen des Sterilisationsmediums durch Einbringen von Wasserstoffperoxid In fein verteilter Form in ein dampf- und/oder gasförmiges Medium, beispielsweise in Luft bzw. in sterile Luft zugeordnet ist.

17. Vorrichtung nach einem der vorhergehenden Ansprüche, **dadurch gekennzeichnet, dass** die Behandlungsstationen in Abhängigkeit von wenigstens einem die Anzahl der je Zeiteinheit behandelten Verschlüsse (2) erfassenden Sensors (35) und/oder in Abhängigkeit von der Transportgeschwindigkeit des Transportelementes (3, 3a) zu- und wegschaltbar sind, und zwar derart, dass die Anzahl der in Transportrichtung (A) aneinander anschließenden für jeweils einen Behandlungsschritt aktivierten Behandlungsstationen (11, 11a) mit steigender Anzahl der je Zeiteinheit behandelten Verschlüsse (2) und/oder mit steigender Transportgeschwindigkeit des Transportelementes (3, 3a) zunimmt und mit sich reduzierender Anzahl der je Zeiteinheit behandelten Verschlüsse (2) oder mit sich reduzierender Transportgeschwindigkeit des Transportelementes (3, 3a) abnimmt.

18. Vorrichtung nach einem der vorhergehenden Ansprüche, **dadurch gekennzeichnet, dass** das Transportelement eine um eine horizontale Achse umlaufend angetriebene Trommel (49) ist, dass der wenigstens eine Behandlungsraum oder die wenigstens eine Behandlungsstrecke innerhalb der Trommel (49) ausgebildet sind, und dass die Trommel (49) an einer Trommelinnenfläche die beispielsweise von Stegen (54) oder Vorsprüngen gebildeten Aufnahmen (55) für die Verschlüsse (2) aufweist.

19. Vorrichtung nach Anspruch 18, **dadurch gekennzeichnet, dass** in dem Trommelinnenraum (51) wenigstens eine mit der Trommel (49) nicht mitbewegte und die Verschlüsse (2) gegen Herausfallen aus den Aufnahmen (55) sichernde Führung (56) vorgesehen ist.

20. Vorrichtung nach Anspruch 18 oder 19, **dadurch gekennzeichnet, dass** auf einem Winkelbereich der Drehbewegung der Trommel (49) wenigstens eine die Behandlungsstrecke bildende Behandlungsstation (11), vorzugsweise mehrere in Drehrichtung (F) der Trommel (49) aufeinander folgende Behandlungsstationen (11) vorgesehen sind.

## Claims

1. A device for sterilising caps, or like closures, (2) for closing bottles or like containers, having at least one treatment station for sterilising the closures (2) and having a circulating transport element (4; 37 - 42; 49) which moves the closures (2) at least on a treatment section and/or in a treatment area for sterilising the closures (2), with the transport element (4; 37 - 42; 49) exhibiting in a transport or circulation direction (A, F) and in sequence a plurality of recipients (43, 55) or baskets (4.1) to each receive at least one closure (2) in such a manner that at least in the direction of transport or movement (A, F) the closures (2) arranged in the recipients (43, 55) or baskets (4.1) assume a defined position and/or orientation relative to the transport element (4; 37 - 42; 49), wherein the transport element (4, 49) is configured to receive the closures (2) from an unsorted quantity and to sort the closures (2) such that the closures exhibit the defined position and/or orientation.

2. The device of claim 1 wherein the at least one circulating transport element (4; 37 - 42; 49) forms part of a transport system (3, 3a) by which the closures (2) are conveyed in a transport direction (A, F) at least through a treatment section having at least one treatment station for sterilising the closures, preferably a treatment section having a plurality of treatment stations (11,11 a) arranged sequentially in transport direction (A, F).

3. The device of any one of the preceding claims wherein the closure recipients or baskets (43, 4.1) are each configured to receive a plurality of closures (2) adjacent to or offset relative to one another square to or at right angles to the transport direction (A).

4. The device of any one of the preceding claims wherein the transport element is a transport belt (4) forming a loop that is closed and can be driven to endlessly circulate, and a plurality of closure recipients or baskets (4.1) is provided sequentially on the transport belt (4) in transport direction (A).

5. The device of claim 4 wherein the transport belt (4) is configured in the manner of a link chain having a plurality of container baskets (4.1) adjacent to one another in transport direction (A) and connected to each another in an articulated manner.

6. The device of claim 4 or 5 wherein the baskets (4.1) are each configured cage-like for as exposed a receiving of the closures (2) as possible.

7. The device of any one of claims 4 to 6 wherein the transport belt (4) passes through a bin (8) for receiving the closures (2), and preferably in the region of a return of the transport belt (4).

8. The device of any one of claims 4 to 7 wherein there are provided along the movement path of the baskets (4.1) or recipients (43) means, for example in the form of mechanical chicanes and/or guides and/or controlled blow jets, by which incorrectly oriented closures (2) are removed from the baskets (4.1) or recipients (43) before such closures (2) reach the treatment stations (11, 11 a) or the treatment section which they constitute.

9. The device of any one of claims 4 to 8 wherein the treatment stations (11, 11a) are provided sequentially in transport direction (A) on at least one side of a loop formed by the transport belt (4), preferably on both sides of the said loop, and again preferably in the region of a return of the transport belt or in the region of a sterilisation drum (9) forming that return.

10. The device of any one of the preceding claims wherein means are provided at the treatment section or at the treatment stations (11, 11 a) to move the closures (2) in the respective basket (4.1) or in the respective recipients (43) square to or at right angles to transport direction (A) and/or to separate them from one another.

11. The device of any one of claims 7 to 10 wherein the bin (8) is configured with a heating device for preheating the closures (2).

12. The device of any one of the preceding claims wherein an ejection device (13) is provided at a closure outlet (12) for the simultaneous discharging of a plurality of closures (2) disposed in at least one basket (4.1) or one recipient (43) preferably into a cap channel (14, 47) connected to the closure outlet (12, 46).

13. The device of any one of the preceding claims wherein are configured at least one treatment station (11) for applying a preferably hot sterilisation medium containing hydrogen peroxide to the closures (2) and at least one treatment station following in transport direction (A) for activating the hydrogen peroxide by heating, for example for applying a heated vaporous and/or gaseous medium, for example for applying heated sterile air and/or for subjecting the closures (2) to radiation energy, for example infrared radiation.

14. The device of claim 13 wherein at least one further treatment station (11, 11a) for preheating the closures (2), for example by applying a heated vaporous and/or gaseous medium, for example heated air or heated sterile air and/or by subjection to a radiation energy, for example infrared radiation, is provided upstream in transport direction (A) of the at least one treatment station for applying the sterilisation medium to the closures (2).

15. The device of any one of the preceding claims wherein a heater (22) is associated with each treatment station (11) or with a group of treatment stations (11, 11 a) or with all treatment stations (11, 11 a) of the device (1, 1 a).

16. The device of any one of the preceding claims wherein a mixer head (24) for generating the sterilisation medium by introducing hydrogen peroxide in finely distributed form into a vaporous and/or gaseous medium, for example air or sterile air, is associated at least with the treatment stations (11) used to apply the sterilisation medium or with a group of such stations.

17. The device of any one of the preceding claims wherein the treatment stations can be activated and deactivated as a function of at least one sensor (35) which counts the number of closures (2) treated per unit of time and/or as a function of the transport speed of the transport element (3, 3a), and in such a way that the number of treatment stations (11, 11a) contiguous in transport direction (A) that are activated per treatment step increases as the number of closures (2) treated per unit of time and/or as the transport speed of the transport element (3, 3a) increases, and decreases as the number of closures (2) treated per unit of time and/or as the transport speed of the transport element (3, 3a) decreases.

18. The device of any one of the preceding claims wherein the transport element is a drum (49) driven to rotate about a horizontal axis, the at least one treatment area or the at least one treatment section is configured inside the drum (49), and the drum (49) exhibits the recipients (55), which are formed for example by ribs (54) or projections, for the closures (2) on an inner face.

19. The device of claim 18 wherein the drum interior (51) is provided with at least one guide (56) which does not move with the drum (49) and protects the closures (2) from falling out of the recipients (55).

20. The device of claim 18 or 19 wherein at least one treatment station (11) forming the treatment section, preferably a plurality of treatment stations (11) disposed sequentially in the direction of rotation (F) of the drum (49), are provided over an angular sector of the rotational motion of the drum (49).

## Revendications

1. Dispositif de stérilisation de capuchons ou d'autres bouchons (2) pour l'obturation de flacons ou d'autres récipients, avec au moins une station de traitement pour la stérilisation des bouchons (2) et avec un élément de transport (4 ; 37 - 42 ; 49) rotatif déplaçant les bouchons (2) au moins sur une trajectoire de traitement et/ou dans une chambre de traitement pour la stérilisation des bouchons (2), l'élément de transport (4 ; 37 - 42 ; 49) comprenant, dans une direction de transport ou de circulation (A, F), plusieurs logements (43, 55) successifs ou corbeilles (4.1) successives, conçus chacun pour le logement d'au moins un bouchon (2) de façon à ce que les bouchons (2) disposés dans les logements (43, 55) ou corbeilles (4.1) présentent, au moins dans la direction de transport ou de mouvement (A, F), une position et/ou une orientation définie par rapport à l'élément de transport (4 ; 37 - 42 ; 49),
**caractérisé en ce que**
l'élément de transport (4, 49) est conçu pour le logement des bouchons (2) à partir d'une quantité non ordonnée et pour le tri des bouchons (2), de façon à ce que les bouchons présentent la position et/ou l'orientation définie.

2. Dispositif selon la revendication 1, **caractérisé en ce que** l'au moins un élément de transport (4 ; 37 - 42 ; 49) rotatif fait partie intégrante d'un système de transport (3, 3a), avec lequel les bouchons (2) sont déplacés dans une direction de transport (A, F), au moins sur une trajectoire de traitement avec au moins une station de traitement pour la stérilisation des bouchons, de préférence sur une trajectoire de traitement avec plusieurs stations de traitement (11, 11a) se succédant dans la direction de transport (A, F).

3. Dispositif selon l'une des revendications précédentes, **caractérisé en ce que** les logements de bouchons ou les corbeilles (43, 4.1) sont conçus chacun pour le logement de plusieurs bouchons (2) s'enchaînant entre eux de manière transversale ou perpendiculaire à la direction de transport (A) ou décalés entre eux.

4. Dispositif selon l'une des revendications précédentes, **caractérisé en ce que** l'élément de transport est une bande de transport (4) formant une boucle fermée et entraînable en rotation sans fin, et **en ce qu'**une pluralité de logements de bouchons ou de corbeilles (4.1) se succédant sur la bande de transport (4) dans la direction de transport (A) est prévue.

5. Dispositif selon la revendication 4, **caractérisé en ce que** la bande de transport (4) est conçue comme une chaîne à maillons avec plusieurs corbeilles de récipients (4.1) s'enchaînant dans la direction de transport (A) et reliées entre elles de manière articulée.

6. Dispositif selon la revendication 4 ou 5, **caractérisé en ce que** les corbeilles (4.1) sont conçues sous la forme de cages pour un logement le plus libre possible des bouchons (2).

7. Dispositif selon l'une des revendications 4 à 6, **caractérisé en ce que** la bande de transport (4) est guidée par un accumulateur (8) pour le logement des bouchons (2), de préférence au niveau d'un renvoi de la bande de transport (4).

8. Dispositif selon l'une des revendications 4 à 7, **caractérisé en ce que**, sur la piste de déplacement des corbeilles (4.1) ou des logements (43), sont prévus des moyens, par exemple sous la forme de chicanes et/ou de guidages mécaniques et/ou de buses de soufflage contrôlées, avec lesquels les bouchons (2) mal orientés peuvent être retirés des corbeilles (4.1) ou des logements (43) avant que ces bouchons (2) atteignent les stations de traitement (11, 11a) ou la trajectoire de traitement constituée par celles-ci.

9. Dispositif selon l'une des revendications 4 à 8, **caractérisé en ce que** les stations de traitement (11, 11a) se succèdent dans la direction de transport (A) sur au moins un côté d'une boucle formée par la bande de transport (4), de préférence sur les deux côtés de cette boucle, de préférence au niveau d'un renvoi de la bande de transport ou au niveau d'un tambour de stérilisation (9) formant ce renvoi.

10. Dispositif selon l'une des revendications précédentes, **caractérisé en ce que**, sur la trajectoire de traitement ou sur les stations de traitement (11, 11a), sont prévus des moyens pour déplacer et/ou éloigner entre eux les bouchons (2) dans la corbeille (4.1) correspondante ou dans les logements (43) correspondants de manière transversale ou perpendiculaire à la direction de transport (A).

11. Dispositif selon l'une des revendications 7 à 10, **caractérisé en ce que** l'accumulateur (8) est muni d'un dispositif de chauffage pour le préchauffage des bouchons (2).

12. Dispositif selon l'une des revendications précédentes, **caractérisé en ce que**, sur une sortie de bouchons (12), se trouve un dispositif d'éjection (13), pour la distribution simultanée de plusieurs bouchons (2) disposés dans au moins une corbeille (4.1) ou un logement (43), de préférence dans un canal de capuchons (14, 47) succédant à la sortie de bouchons (12, 46).

13. Dispositif selon l'une des revendications précédentes, **caractérisé en ce qu'**au moins une station de traitement (11) est conçue pour l'application d'un fluide de stérilisation, de préférence très chaud, contenant du peroxyde d'hydrogène, sur les bouchons (2) et au moins une station de traitement suivante dans la direction de transport (A) est conçue pour l'activation du peroxyde d'hydrogène par chauffage, par exemple pour la distribution d'un fluide chauffé sous forme de vapeur et/ou de gaz, par exemple pour la distribution d'un air chauffé stérile et/ou pour l'alimentation des bouchons (2) en énergie de rayonnement, par exemple avec un rayonnement infrarouge.

14. Dispositif selon l'une la revendication 13, **caractérisé en ce que**, avant l'au moins une station de traitement conçue pour l'alimentation des bouchons (2) avec le fluide de stérilisation dans la direction de transport (A), au moins une autre station de traitement (11, 11a) est prévue pour le préchauffage des bouchons (2), par exemple par l'alimentation avec un fluide chauffé sous forme de vapeur et/ou de gaz, par exemple avec de l'air chauffé ou de l'air stérile chauffé et/ou par l'alimentation avec une énergie de rayonnement, par exemple un rayonnement infrarouge.

15. Dispositif selon l'une des revendications précédentes, **caractérisé en ce qu'**à chaque station de traitement (11) ou à un groupe de stations de traitement (11, 11 a) ou à toutes les stations de traitement (11, 11 a) du dispositif (1, 1a), correspond un réchauffeur (22).

16. Dispositif selon l'une des revendications précédentes, **caractérisé en ce qu'**à au moins les stations de traitement (11) servant à distribuer le fluide de stérilisation ou à un groupe de ces stations, correspond une tête de mélange (24) pour la production du fluide de stérilisation par l'apport de peroxyde d'hydrogène sous forme finement pulvérisée dans un fluide sous forme de vapeur et/ou de gaz, par exemple dans l'air ou dans l'air stérile.

17. Dispositif selon l'une des revendications précédentes, **caractérisé en ce que** les stations de traitement peuvent être activées et désactivées en fonction d'au moins un capteur (35) déterminant le nombre de bouchons (2) traités par unité de temps et/ou en fonction de la vitesse de transport de l'élément de transport (3, 3a), de telle sorte que le nombre de stations de traitement (11, 11a) activées s'enchaînant dans la direction de transport (A) pour une étape de traitement augmente lorsque le nombre de bouchons (2) traités par unité de temps augmente et/ou lorsque la vitesse de transport de l'élément de transport (3, 3a) augmente et diminue lorsque le nombre de bouchons (2) traités par unité temps diminue ou lorsque la vitesse de transport de l'élément de transport (3, 3a) diminue.

18. Dispositif selon l'une des revendications précédentes, **caractérisé en ce que** l'élément de transport est un tambour (49) entraîné en rotation autour d'un axe horizontal, **en ce que** l'au moins une chambre de traitement ou l'au moins une trajectoire de traitement se trouve à l'intérieur du tambour (49) et **en ce que** le tambour (49) comprend, sur une surface interne, les logements (55) pour les bouchons (2), constitués par exemple de nervures (54) ou de saillies.

19. Dispositif selon la revendication 18, **caractérisé en ce que**, dans l'espace intérieur du tambour (51), se trouve au moins un guidage (56) ne se déplaçant pas avec le tambour (49) et qui empêche les bouchons (2) de tomber hors des logements (55).

20. Dispositif selon la revendication 18 ou 19, **caractérisé en ce que**, sur une zone angulaire du mouvement de rotation du tambour (49), se trouve au moins une station de traitement (11) formant la trajectoire de traitement, de préférence plusieurs stations de traitement (11) se succédant dans la direction de rotation (F) du tambour (49).
